# EUROPEAN PATENT SPECIFICATION

(11) **EP 1 372 610 B1**
(45) Date of publication and mention of the grant of the patent: **09.05.2012**
(21) Application number: 02745193.9
(22) Date of filing: 16.03.2002
(51) Int. Cl.: A61K 9/50

(54) **POWDER INHALER FORMULATIONS**
PULVERINHALATOR-FORMULIERUNGEN
FORMULATIONS EN POUDRE POUR INHALATEUR

(30) Priority: 21.03.2001 GB 0107106
(43) Date of publication of application: 02.01.2004
(73) Proprietor: Boehringer Ingelheim Pharma GmbH & Co.KG, 55216 Ingelheim am Rhein (DE)
(72) Inventor: BECHTOLD-PETERS, Karoline, 88400 Biberach-Rissegg (DE); NGUYEN, Hanh, Alnwick, Northumberland NE66 1XX (GB); ROWLEY, Geoffrey, Sunderland,Tyne and Wear SR2 9DX (GB)
(86) International application number: PCT/EP2002/002948
(87) International publication number: WO 2002/080884

(56) References cited:
- WO-A-00/57881
- WO-A-92/08447
- WO-A-92/09322
- US-A- 3 082 154
- US-A- 4 533 542
- US-A- 4 540 602
- CASSIDY O ET AL: "The effect of surface modification on electrostatic charge of a particulate solid." EUROPEAN JOURNAL OF PHARMACEUTICAL SCIENCES, vol. 4, no. SUPPL., 1996, page S65 XP002232704 Third European Congress of Pharmaceutical Sciences;Edinburgh, Scotland, UK; September 15-17, 1996 ISSN: 0928-0987
- NGUYEN H ET AL: "The potential of surface modification to improve wetting, dissolution and electrostatic charge of a hydrophobic drug." JOURNAL OF PHARMACY AND PHARMACOLOGY, vol. 49, no. SUPPL. 4, 1997, page 41 XP009005828 134th Meeting of the British Pharmaceutical Conference;Scarborough, England, UK; September 15-18, 1997 ISSN: 0022-3573
- CASSIDY O E ET AL: "Surface modification and electrostatic charge of polystyrene particles." INTERNATIONAL JOURNAL OF PHARMACEUTICS. NETHERLANDS 25 MAY 1999, vol. 182, no. 2, 25 May 1999 (1999-05-25), pages 199-211, XP001145533 ISSN: 0378-5173
- CASSIDY, O.E. AND ROWLEY, G.: "The effect of drug surface modification by adsorbed polymer on electrostatic charge" PROC. PHARM. TECH. CONF., vol. 16, no. 3, 1997, pages 5-11, XP009005985
- ROWLEY G ET AL: "The development of a particle carrier technique to aid the investigation of electrostatic charging properties of micronized pharmaceutical powders." JOURNAL OF PHARMACY AND PHARMACOLOGY, vol. 47, no. 12B, 1995, page 1100 XP009005825 British Pharmaceutical Conference 1995: Science Proceedings of the 132nd Meeting;Warwick, England, UK; September 15-18, 1995 ISSN: 0022-3573
- CARTER P A ET AL: "Electrostatic charge on inhaled powers." EUROPEAN JOURNAL OF PHARMACEUTICAL SCIENCES, vol. 6, no. SUPPL. 1, August 1998 (1998-08), page S63 XP002232941 Fourth European Congress of Pharmaceutical Sciences;Milan, Italy; September 11-13, 1998 ISSN: 0928-0987

## Description

The present invention relates to new methods for the surface modification of powders. Furthermore the present invention relates to new, improved pharmaceutical dosage forms obtainable by the new methods for surface modification of drugs according to the invention and to the use of these pharmaceutical dosage forms within dry powder inhalation devices (DPI).

### Background of the invention

Active substances for dry powder inhalation are often prepared by micronization or by spray drying to have an aerodynamic particle size of approximately 5 µm or less enabling lung deposition. Such powders present difficulties in manufacture and handling as well as in dispensing these powders during application due to particle agglomeration, cohesion and adhesion to manufacturing equipment, inhaler devices and container materials.

It is the object of the present invention to provide for new pharmaceutical dosage forms that are producible and applicable without displaying the drawbacks of conventional micronized or spray-dried powders for inhalation. In particular it is the object of the invention to provide for new pharmaceutical dosage forms being characterized by reduced electrostatic chargeability of the microfine active substances thereby improving powder flow properties during the manufacture of DPIs and improving powder dispensing and dispersion properties during application. Moreover, it is the object of the present invention to provide for a process of manufacture of these powders for inhalation.

### Description of the invention

Surprisingly it has been found, that the aforementioned object of the invention is solved by an improved pharmaceutical dosage form for the use in a dry powder inhalation device (DPI) which comprises (a) at least one micronized or microfine solid active ingredient, which is soluble in water, and which is selceted from among ipratropium, tiotropium, fenoterol and formoterol (b) optionally a solid, pharmaceutically acceptable carrier excipient, which dilutes the active ingredient (a), (c) a fatty acid sorbitan ester or a PEG ether thereof, characterized in that the fatty acid sorbitan ester or PEG ether thereof (c) coats at least partially the surface of (a), or of the agglomerate formed by (a) and (b).

The micronized or microfine solid active ingredients are drugs for medical or diagnostic use. They are generally selected from those medicaments that are applicable via inhalation. Preferably they may be selected from the group consisting of anti-COPD-agents, anti-asthmatics, anti-migraine agents, anti-infective agents, anti-pain-agents, proteoglycans, therapeutic proteins, peptides and genes. Preferred active ingredients according to the invention are selected from the group consisting of beta-agonists such as Fenoterol, Formoterol and Salmeterol, anticholinergic drugs such as Ipratropium, Oxitropium, and Tiotropium, or combinations of beta-agonists and anticholinergics such as Tiotropium + Formoterol or Salmeterol, interferons such as interferon-alpha, interferon-beta, interferon-gamma or interferon-omega, cytokines such as interleukins and their antagonists or receptors, peptide hormones and analogues such as LHRH analogues, growth hormones and analogues, colony stimulating factors, erythropoietin, TNFs, vaccines, blood factors, enzymes, parathyroid hormone, calcitonin, insulin, antibodies such as antibodies to treat immune diseases, virus infections or lung cancer, alpha-1-antitrypsin, proteoglycans such as heparin or low molecular weight heparins, genes, anti-migraine drugs such as BIBN 4096, wherein Ipratropium, Tiotropium, Fenoterol, Salmeterol, Formoterol, or combinations of Tiotropium with Formoterol or Salmeterol, BIBN 4096, interferons, interleukin receptors and RSV-antibodies are the most preferred active ingredients.

Within the contents of this invention a reference to the aforementioned active ingredients is to be understood as reference to the active ingredients optionally in the form of their pharmaceutically acceptable acid addition salts, in the form of their solvates and hydrates.

The pharmaceutically acceptable acid addition salts are selected from the group consisting of hydrochloride, hydrobromide, sulfate, phosphate, methansulfonate, acetate, fumarate, lactate, citrate, tartrate and maleate. Preferred acid addition salts are selected form the group consisting of hydrochloride, hydrobromide, sulfate, phosphate and methansulfonate. More preferred acid addition salts are selected from the group consisting of hydrochloride, hydrobromide and methansulfonate.

If the active ingredient is selected from the group consisting of Ipratropium, Oxitroprium and Tiotropium reference to these ingredients is to be understood as reference to their salts selected from the group consisting of chloride, bromide, iodide, methansulfonate, para-toluenesulfonate or methylsulfate. In the aforemenetioned salts the active ingredients Ipratropium, Oxitroprium and Tiotropium represent kations. Preferred salts of Ipratropium, Oxitropium and Tiotropium are selected from the group consisting of chloride, bromide, iodide and methansulfonate, more preferred are methansulfonate and bromide, the latter one being most preferred.

The active ingredients used for the preparation of the pharmaceutical dosage forms according to the invention can optionally form solvates or hydrates. Accordingly, the term active ingredient not only relates to the salts and acid addition salts as specified hereinbefore, but embraces optionally existing solvates or hydrates thereof. In case of the preferred active ingredient Tiotropiumbromide the monohydrate thereof is of particular interest.

Within the contents of this invention acceptable carrier or, in the case of spray dried active ingredients, encapsulation excipients are selected from the group consisting of monosaccharides (e.g. glucose or arabinose), disaccharides (e.g. lactose, trehalose, sucrose, maltose), oligo- and polysaccharides (e.g. dextranes, hydroxyethyl cellulose), polyalcohols (e.g. sorbit, mannitol, xylit), salts (e.g. sodium chloride, calciumcarbonate), polyesters (e.g. polylactides and their copolymers), polyethers (e.g. PEG), sugar esters and ethers, polyvinyl derivatives (e.g. polyvinylalcohol) or mixtures thereof. Preferred acceptable carrier excipients are selected from mono- or disaccharides, especially lactose and glucose, optionally in the form of their hydrates. Of particular interest according to the invention are lactose-monohydrate and anhydrous glucose. Of particular interest as encapsulating agents are hydroxyethyl starch, trehalose, mannitol and lactose monohydrate or mixtures of mannitol and sucrose.

The average geometric particle size of the optionally added acceptable carrier excipients is in the range of 2 - 100µm, preferably 4-60 µm, more preferably 6-40 µm, most preferably 8-35 µm. Of particular interest according to the invention are for example the following carrier excipients: Lactose monohydrate 200 mesh, optionally in mixture with micronized lactose, and glucose anhydrous 35 µm, optionally in mixture with micronized anhydrous glucose.

The average geometric particle size of the drug substance in line with this patent is 0.5 - 25 µm, preferably 1 - 20 µm, more preferably 1-15 µm. The average mass median aerodynamic diameter (MMAD) of the drug substance in this patent is targeted to be 0.5-15 µm, preferably 0.5-10 µm, more preferably 0.5 - 8 µm.

According to this invention, the term average geometric particle size is defined as the value in µm at which 50% of the particles as determined from the volume distribution of the particles by laser diffraction (dry suspension method) are smaller than or equal to this value. The MMAD in accordance with this patent is measured using appropriate devices such as cascade impactors or impingers as described and defined in the current pharmacopeias (e.g.: European Pharmacopoeia - Supplement 2001, pages 113-124 and 1657 - 1661).

According to the invention the the fatty acid or fatty alcohol derivatives are sorbitol derivatives, optionally containing polyethylene glycol ether groups, particularly they are selected from the group consisting of sorbitan mono-oleate, sorbitan trioleate, sorbitan monostearate, sorbitan tristearate, sorbitan monolaurate, sorbitan trilaurate, sorbitan monomyristate, sorbitan trimyristate, sorbitan monopalmitate, sorbitan tripalmitate, preferred PEG derivatives are PEG sorbitan monolaurate, PEG sorbitan monopalmitate, PEG sorbitan monostearate, PEG sorbitan tristearate, PEG sorbitan mono-oleate and PEG sorbitan trioleate. Preferred sorbitol derivatives are sorbitan mono-oleate , sorbitan trioleate sorbitan monostearate, sorbitan tristearate, PEG sorbitan monolaurate and PEG sorbitan mono-oleate, most preferred being sorbitan mono-oleate, sorbitan monostearate, sorbitan tristearate and PEG sorbitan mono-oleate.

Within the contents of the invention the term pharmaceutical dosage form is to be regarded as being equivalent to the term powder for inhalation.

The amounts of fatty acid or fatty alcohol derivative or poloxamer relative to the drug substance or - if carriers or encapsulating agents are present - relative to the drug substance plus excipient complex, i.e. the drug substance-excipient agglomerate or mixture or microcapsule, are in the range of 0.001 - 200% w/w, preferably 0.002 - 100% w/w, more preferably 0.01 - 50% w/w. Drug substance and surface modifying component together constitute 0.02 - 100% w/w, preferably 0.05 -100% w/w, more preferably 0.1 - 100% w/w of the pharmaceutical dosage form.

The pharmaceutical dosage form according to the invention, is obtainable via processes of surface modification, involving the physical adsorption of a fatty acid or alcohol derivative or poloxamer (c) from solution or dispersion onto the surface of a drug (a), present as an insoluble particulate dispersion or by spray drying a solution or dispersion of the drug containing said fatty acid or alcohol derivative or poloxamer or by intensively physically mixing a powder containing the microfine drug with the fatty acid or alcohol derivative or poloxamer.

One process (process A) according to the invention comprises the steps of
(i) preparation of a solution or dispersion of components (c)in a solvent, in which components (a) and optionally a carrier (b) are insoluble;
(ii) adsorption of components (c) to the surface of (a) and optionally (b) until equilibration;
(iii) separation of the dosage form by filtration and/or centrifugation, and
(iv) optionally drying of the resulting dosage form.

Another process (process B; spray drying process) according to the invention comprises the steps of:
(i) dissolving or dispersing components (a) and (c) in the solvent, optionally also adding encapsulating agents (d),
(ii) spray drying the solution or dispersion in a spray dryer under appropriate conditions resulting in microfine particles according to the particle size range described above
(iii) harvesting the spray dried particles in the cyclone or in the filter
(iv) optionally drying the particles to reach the wanted moisture content
(v) and finally optionally diluting the powder by addition of a carrier substance (b).

Another process (process C) according to the invention comprises the steps of:
(i) intensively mixing a powder containing the microfine drug substance (a), optionally also drug carrier (b), using standard mixing machines such as a Diosna mixer or a Lödige mixer,
(ii) either adding before start of the mixing process or, preferentially, during the mixing process components (c) to the powder and
(iii) running the mixing process for a while to enable that components (c) coat the surface of components (a) and optionally (b).

Another aspect of the invention relates to the processes of preparation of a pharmaceutical dosage form as described hereinbefore. Another aspect of the invention relates to a pharmaceutical dosage form obtainable via to the aforementioned process.

In the first process according to the invention (process A), the active substances are water soluble and thus a non-aqueous solvent, preferably a water-immiscible organic solvent, was required for the adsorbate. Therefore, the solvent for step (i) in the first process (process A) is preferably a C₃-C₁₂ alkane or a C₃-C₁₂ cycloalkane, more preferably a C₅-C₈ alkane or a C₅-C₈ cycloalkane. The most preferred solvent is n-hexane or cyclohexane.

In the second process according to the invention (process B) the solvent for step (i) in needs not to be a solvent in which for instance component (a) is insoluble. The solvent is preferably selected from water, aqueous buffer-solutions like for instance phosphate-buffer solutions, alcohols like for instance methanol, ethanol or isopropanol, C₃-C₁₂ alkanes, C₃-C₁₂ cycloalkanes or mixtures thereof. Preferred solvents for step (i) in process B are selected from water, aqueous buffer-solutions like phosphate-buffer solutions, alcohols and mixtures thereof, water and phosphate-buffer solutions being most preferred.

The concentration of the fatty acid or alcohol derivative or poloxamer in the solvent according to process A can vary from 20 mg *l* L to 10,000 mg / L, is preferably between 100 mg to 8,000 mg / L, more preferably between 200 mg and 5,000 mg / L, the most preferred concentration being 2000 mg / L.

In processes B and C the amount of fatty acid or fatty alcohol derivative or poloxamer added relative to the total solids is in the range of 0.001 to 50 % w/w, preferably between 0.005 and 10 % w/w, most preferred between 0.01 and 5 % w/w.

In the processes according to the invention the drug substance is added in concentrations between 0.001% and 50%, preferably between 0.1% and 20%, the most preferred concentration is 2%, i.e. 4 g / 200 ml.

From the aforementioned processes A, B, and C processes B and C are of particular interest, especially for processes in technical scale.

The pharmaceutical dosage forms display a variety of surprising and unexpected advantages and are therefore superior over conventional micronized and microfine powders for inhalation. By the surface modification of the active substances via adsorption of or coating by or intensive mixing with fatty acid derivatives the following effects proved to be of extraordinary significance:
(a) reduction of electrostatic charge acquisition by triboelectrification during pharmaceutical processing and during handling/drug administration,
(b) reduction of adhesion to contact surfaces,
(c) improvement of powder flow during pneumatic transport,
(d) improvement of drug content uniformity during mixing of actives with excipient carriers in DPI formulations and
(e) improvement of inhalation properties of powders.

The methods generally provide for
- the reduction of electrostatic charge acquisition by triboelectrification during pharmaceutical processing and during handling/drug administration, and
- the reduction of adhesion to contact surfaces.

It is to be understood that these methods, even though being preferably applicable for the preparation and application of inhalation powders, are not limited to these powders.

Accordingly, a further aspect generally relates to a method for the reduction of electrostatic charge acquisition by triboelectrification during pharmaceutical processing and during handling/drug administration, characterized in that a surface modification involving the physical adsorption of a fatty acid or alcohol derivative or poloxamer from solution or dispersion onto the surface of a drug present as an insoluble particulate dispersion in the solution or the coating of the dissolved or dispersed drug by a fatty acid or alcohol derivative or poloxamer using spray drying or the intensve mixing of a drug containing powder with a fatty acid or alcohol derivative or poloxamer is conducted,

Another aspect generally relates to a method for the reduction of adhesion to contact surfaces, characterized in that a surface modification involving the physical adsorption of a fatty acid or alcohol derivative or poloxamer from solution or dispersion onto the surface of a drug present as an insoluble particulate dispersion in the solution or the coating of the dissolved or dispersed drug by a fatty acid or alcohol derivative or poloxamer using spray drying or the intensive mixing of a drug containing powder with a fatty acid or alcohol or poloxamerderivative is conducted.

Another aspect relates to a method for the improvement of powder flow during pneumatic transport, characterized in that a surface modification involving the physical adsorption of a fatty acid or alcohol derivative or poloxamer from solution or dispersion onto the surface of a drug present as an insoluble particulate dispersion in the solution or the coating of the dissolved or dispersed drug by a fatty acid or alcohol derivative or poloxamer using spray drying or the intensve mixing of a drug containing powder with a fatty acid or alcohol derivative or poloxamer is conducted.

Another aspect relates to a method for the improvement of drug content uniformity during mixing of actives with excipient carriers in DPI formulations, characterized in that a surface modification involving the physical adsorption of a fatty acid or alcohol derivative or poloxamer from solution or dispersion onto the surface of a drug present as an insoluble particulate dispersion in the solution or the coating of the dissolved or dispersed drug by a fatty acid or alcohol derivative or poloxamer using spray drying or the intensive mixing of a drug containing powder with a fatty acid or alcohol derivative or poloxamer is conducted.

Another aspect relates to a method for the improvement of inhalation properties of powders, characterized in that a surface modification involving the physical adsorption of a fatty acid or alcohol derivative or poloxamer from solution or dispersion onto the surface of a drug present as an insoluble particulate dispersion in the solution or the coating of the dissolved or dispersed drug by a fatty acid or alcohol derivative or poloxamer using spray drying or the intensve mixing of a drug containing powder with a fatty acid or alcohol derivative or poloxamer is conducted.

The advantages of the inhalation powders (pharmaceutical dosage forms) over conventional inhalation powders mentioned before are discussed and demonstrated in more detail below.

In the processing of micronized or microfine active substances for DPI, it is common to subject the powder to a sieving process in order to remove large agglomerates prior to mixing with the carrier particles used in the DPI formulation. Experimental evidence shows that sieved untreated samples have greater electrostatic charge acquisition by a process of triboelectrification against a contact surface of stainless steel in a cyclone separator. The experimental method for electrostatic charge determinations that was applied is outlined in more detail below.

Comparison of sieved samples of unmodified active and active modified by the adsorption process shows considerable differences In acquired charge. The method applied for the preparation of sieved powder samples is outlined In detail below.

### Brief description of the drawings:

Figure 1: Mean specific charge of micronized Fenoterol generated during triboelectrification in a stainless steel cyclone with or without sieving and with and without organic solvent / antistatic agent treatment;
Figure 2: Mass of micronized Fenoterol (1 g samples) transported to the Faraday well during triboelectrification in a stainless steel cyclone with or without sieving and with and without organic solvent / antistatic agent treatment;
Figure 3: Mean specific charge of micronized Tiotropium generated during triboelectrification in a stainless steel cyclone with or without sieving and with and without organic solvent / antistatic agent treatment;
Figure 4: Mass of micronized Tiotropium (1 g samples) transported to the Faraday well during triboelectrification in a stainless steel cyclone with or without sieving and with and without organic solvent / antistatic agent treatment;
Figure 5: Mean specific charge after mixing in Turbula mixer (Fenoterol and Ipratropium);
Figure 6: Mean specific charge after mixing in Turbula mixer (Tiotropium and Oxitropium);

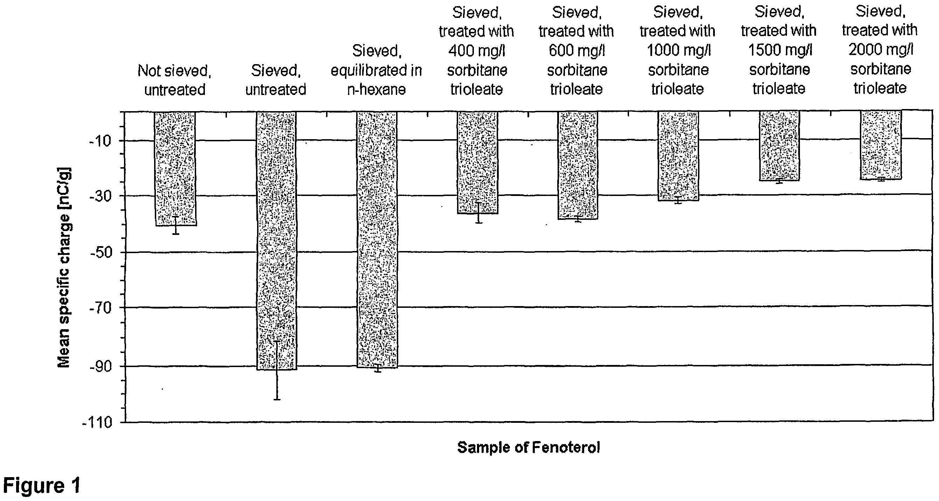

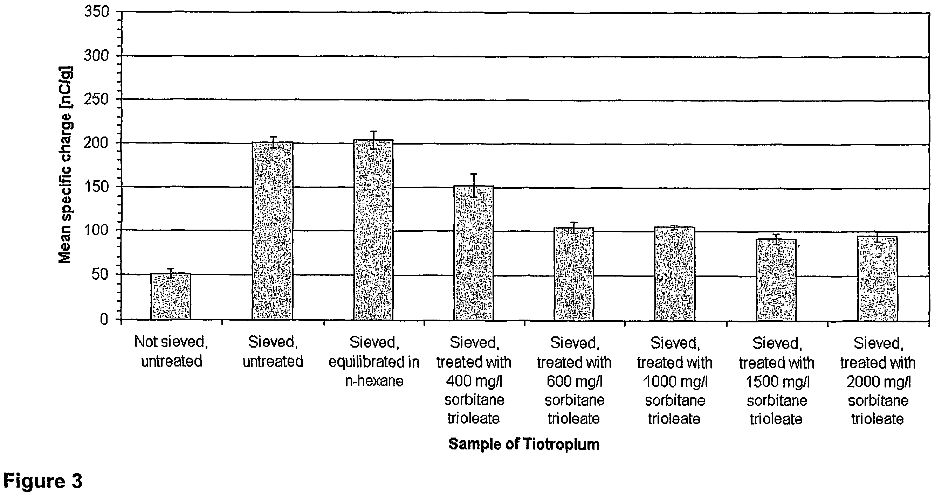

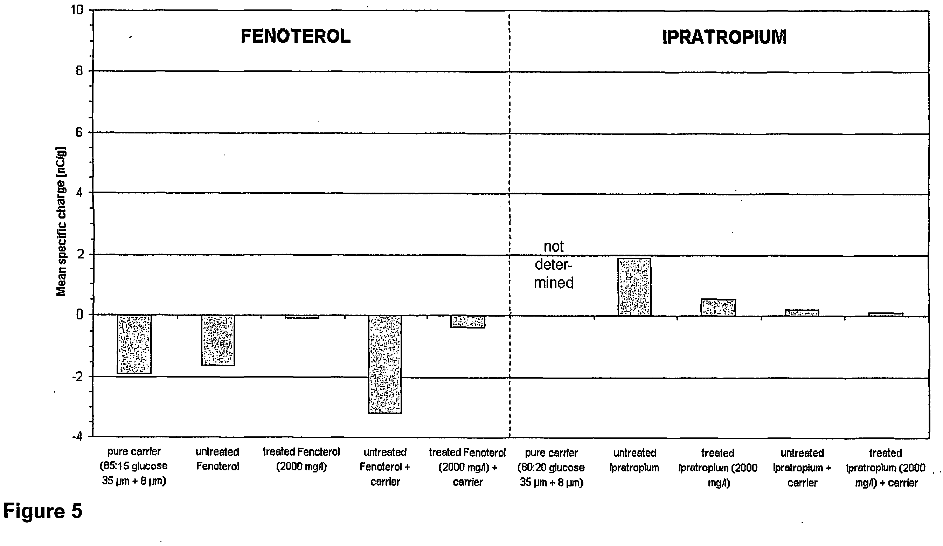

Figure 1 provides specific charge values of -40 and -92 nC g⁻¹ for unsieved and sieved fenoterol respectively and the charge values in figure 3 for unsieved and sieved tioptropium were +52 and +201 nC g⁻¹ respectively. Figures 1 and 3 show that treatment of the active substances with sorbitan trioleate reduces charge acquistion of sieved samples when using the same process of triboelectrification. An example from these data in figures 3 and 5 shows the mean charge values for the drugs fenoterol and tiotropium when treated at a concentration of 600mg l⁻¹ of sorbitan trioleate in hexane. Sieved samples of the treated fenoterol and tiotropium had mean charge values of -38.4 and +104 nC g⁻¹ respectively, after triboelectrification in the cyclone apparatus. These data show that charge acquisition for sieved samples can be reduced by surface modification.

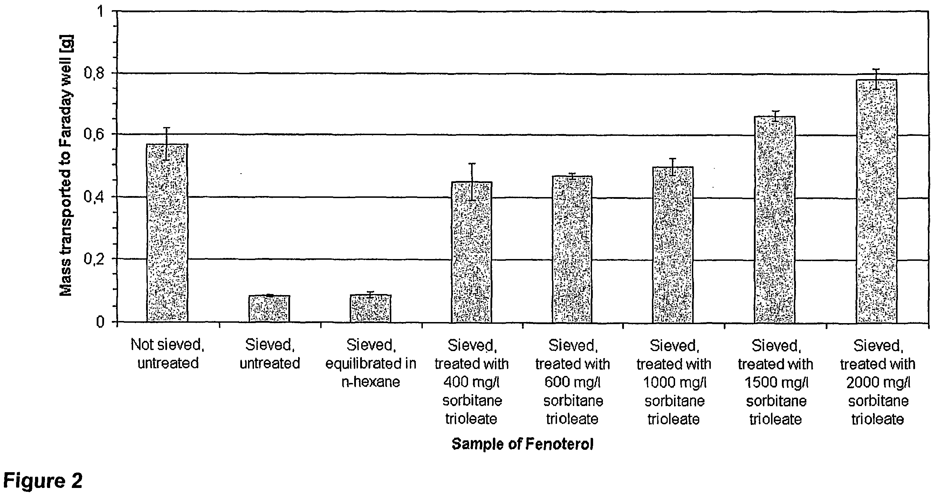

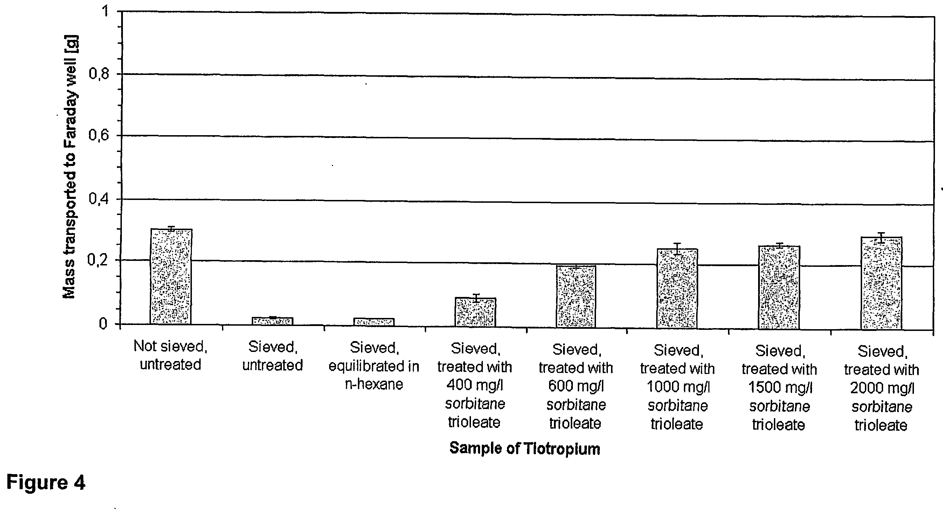

Experimental results show that sieving also adversely affects bulk powder properties of the active substances, including adhesion to contact surfaces and pneumatic flow. Figures 2 and 4 provide mass transfer values of powder through the cyclone apparatus by pneumatic conveyance during triboelectrification experiments. Ideally, 100% w/w of the original sample (1 g) should pass through the apparatus and this would indicate good flow and non-adhesion.

Figures 2 and 4 provide values of mass transfer of 0.083 and 0.025g (8.3 and 2.5 %w/w) for sieved, untreated fenoterol and tiotropium respectively. Treatment of the actives by surface modification with sorbitan trioleate increased the mass transfer values to an extent that was dependent upon treatment concentration. Figure 2 shows increases in mass transfer to between 0.45-0.78g (45-78% w/w) for fenoterol and in figure 4 the values increase to between 0.092- 0.29 g (9.2- 29% w/w) for tiotropium.

Visual inspection of the steel contact surface showed that powder adhesion was considerably less for surface modified actives. In addition, the adhered treated samples were very easily removed, whereas untreated actives were firmly adhered and very difficult to remove.

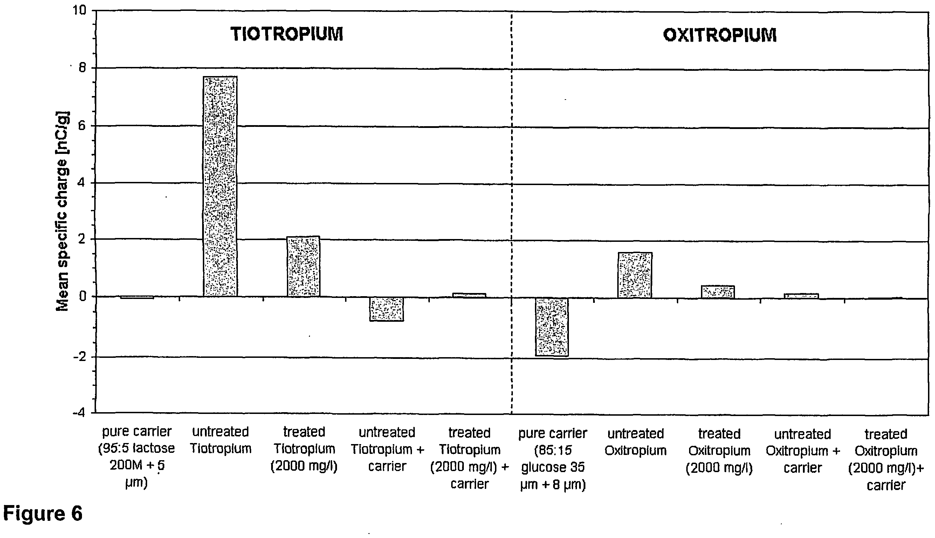

Triboelectrification of powders occurs during mixing processes. Figures 5 and 6 show values for charge acquisition for powder samples of, (a) carrier excipients, (b) untreated and treated actives and (c) DPI formulations of untreated and treated actives. The results in these figures show that the treatment by adsorption of sorbitan trioleate reduces charge acquisition of both the unformulated and formulated actives during mixing in a steel mixing vessel of a turbula mixer( for method see experimental part III). Untreated fenoterol in a DPI formulation with glucose as carrier had a mean specific charge of -3.2 nC g⁻¹, whereas the formulation containing treated drug had a value of -0.35 nC g⁻¹ (figure 5). Tiotropium (untreated) in DPI formulation with lactose as carrier had a mean charge value of -0.78 nC g⁻¹ and the formulation containing treated drug had a value of 0.15 nC g⁻¹ (figure 6).

DPI formulations containing untreated and treated actives were prepared by mixing in a steel vessel of a turbula mixer and 20 random samples from each mix were analysed for the active component. The methodology applied is outlined in detail below. The mean drug content and coefficient of variation (cv) values in table 1 show that the treatment of tiotropium with sorbitan trioleate improves the mixing quality and hence the drug content uniformity.

**Table 1: Mean drug content and coefficient of variation values for DPI formulations prepared in a turbula mixer:**

| **DPI formulation** | **Mean drug content (mg)** | **cv (%)** |
|---|---|---|
| Untreated tiotropium | 0.24 | 45.8 |
| Treated tiotropium (sorbitan trioleate at 2000 mg / L concentration) | 0.22 | 4.5 |
| Untreated fenoterol | 2.1 | 30.9 |
| Treated fenoterol (sorbitan trioleate at 2000 mg / L concentration) | 2.0 | 4.0 |

The effect of different sorbitan derivatives on charge and mass transfer is summarized in table 2 for tiotropium. In all cases, the charge value acquired by triboelectrification in the cyclone apparatus is lower than for untreated tiotropium. The mass transfer values indicate that sorbitan mono-oleate is the most effective derivate for charge reduction and there is little difference in effectiveness between the stearate derivatives.

**Table 2: Mean charge nC g⁻¹ (cv%), mass transfer (%w/w) (cv%) for sieved samples of untreated tiotropium and tiotropium treated with sorbitan derivatives at 600 mg l⁻¹**

| **Sorbitan derivative** | **Mean charge nC g⁻¹** | **Mean mass transfer %** |
|---|---|---|
| Mono-oleate | +39.6 (4.6) | 53 (3.8) |
| Trioleate | +104.5(5.9) | 19.4(3.1) |
| Monostearate | +75.1 (1.1) | 33(9.1) |
| Tristearate | +70.1 (3.1) | 17 (11.8) |
| Untreated tiotropium | +201 (3.2) | 2.5 (8.0) |

### Experimental Part:

### I. Electrostatic Charge Determinations

### Triboelectrification in a cyclone separator

Electrostatic charge of powder samples was investigated using a cyclone apparatus linked to a Faraday well and force compensation load cell to measure charge and mass simultaneously. 1 g samples of powder were transported through the apparatus using dry compressed air (rh < 10%) at 8 m s⁻¹ for triboelectrification against a stainless steel surface.

The charge Q (nC) and mass M (g) values were used to calculate the specific charge Q/M (nC g⁻¹) at the completion of each experimental run. The results are mean values with coefficient of variation values for 5 replicates. The mass of material entering the Faraday well was used to quantify the mass transport through the apparatus and this was used to assess the flow and adhesion characteristics of the powder. In addition, the amount of material adhered to the cyclone wall was estimated visually and rated on a scale from 0 (no adhesion) to 3 (extensive adhesion).

### Triboelectrification in a Turbula mixer

The electrostatic charge of the drug/carrier powder mixes (5g) was undertaken after mixing in a stainless steel cylindrical vessel, agitated at 100 rpm for 10 minutes on a Turbula mixer under ambient conditions, by pouring the sample into a Faraday well. The mass of powder entering the Faraday well was recorded to determine the specific charge. In addition, the difference between the mass of powder in the mixing vessel and that in the Faraday well was used to quantify the amount of adhesion to the mixer vessel wall. The mean specific charge, and coefficient of variation values for 3 replicates are reported.

### II. Preparation of sieved powder samples:

Approximately 10 g of drug powder samples were placed in a 60M (250 µm) sieve and agitated using a sieve shaker (Glen Creston, 47-300) with an oscillation amplitude regulator at setting 20 for 20 minutes. Sieved powder samples were stored in glass jars and then kept in a desiccator for a week prior to charge investigations in the cyclone

### III: Effects of mixing

### Effect on charging

The untreated and treated active substances were mixed with carrier excipient in a ratio selected from the range of drug/carrier compositions used in dry powder inhaler formulations. A carrier blend of coarse and micronized carrier was prepared in a turbula mixer for 10 minutes at 100rpm. The active substance (treated or untreated drug) was added and mixed for further 10 minutes prior to charging measurements.

### Effect of treatment on drug content and uniformity

The untreated and treated drugs were mixed with carrier excipient as follows.
5.2036 g lactose 200M:
0.2739 g micronized lactose:
0.0225 g untreated or treated Tiotropium

4.4880 g glucose 35 µm:
0.7920 g glucose 15 µm:
0.2200 g untreated or treated Fenoterol (total mixing time reduced to 10 minutes, comprising 5 for carrier blend and 5 for carrier/active blend.)

20 samples, approximately 50mg, were taken at random from each mixed formulation, accurately weighed and dissolved in 20 ml distilled water. Drug concentration in each sample was determined spectrophotometrically at λₘₐₓ 237nm and 276 nm for tiotropium and fenoterol respectively. A modified BP content uniformity was applied (20 samples were examined). The mean drug content and coefficient of variation were calculated.

### IV: Preparation of pharmaceutical dosage forms:

### Starting materials:

The starting materials are unless otherwise specified commercially available or obtainable via convenional methods known in the art.

### Tiotropiumbromide monohydrate:

15.0 kg Tiotropiumbromide are introduced into 25,7 kg water. Th mixture is heated to 80-90°C and stirred at that temperature until a clear solution is obtained. Charcoal (0.8 kg) is introduced into 4.4 kg water and the mixture thus obtained is added to the aforementioned solution of tiotropiumbromide. The obtained reaction mixture is stirred for at least 15 min at 80-90°C and is, subsequently, hot-filtered into another reaction apparatus being preheated to about 70°C. The filter is washed with 8.6 kg of water. The mixture thus obtained is cooled to about 20-25°C (3-5°C per 20 minutes). The crystallization is completed by stirring at the aforementioned temperature for at least 1 hour. The crystalline product is isolated and washed with 9 L of cold water (10-15°C) and cold acetone (10-15°C). The crystals are dried for 2 hours at about 25°C under nitrogen. Yield : 13.4 kg tiotropiumbromide monohydrate (86 %).

The crystalline tiotropiumbromide monohydrate thus obtained is micronized according to conventional methods known in the art.

### Preparation of formulation via physical adsorption:

4 g of drug were equilibrated with adsorbate in hexane in a concentration range from 200 to 2 x 10³ mg I in an incubator agitated at 220rpm for 3 hours at 25 ± 0.5°C. The treated drug was filtered using vacuum and dried in a fume cupboard to constant weight at room temperature. Dried treated drugs were lightly milled using a mortar and a pestle.

### Preparation of formulation via spray drying:

Up to 20 g solids including the drug substance, the embedding agent and 0.001 to 2% (w/100 ml) of the fatty acid or alcohol derivative or poloxamer were dissolved or dispersed in water or aqueous buffer solution, e.g. 20 mM phosphate buffer, in an alcohol, a ketone, a hydrocarbon or halogenated hydrocarbon, or in a mixture thereof. The mixture was spray dried using an appropriate spray dryer such as a Büchi Mini SprayDrier, a Niro SDMicro or a Niro Mobile Minor, and harvested from the cyclon or the filter or both. The resulting powder may be vacuum dried at 40°C to reduce residual moisture.

### V. Examples for formulation of pharmaceutical dosage forms prepared in line with this patent:

### Example 1:

4 g Fenoterol hydrobromide are dispersed in an incubator in 200 ml of n-hexane containing 2000 mg/L sorbitan trioleate and agitated at 220rpm for 3 hours at 25 ± 0.5°C. The treated drug is filtered using vacuum and dried in a fume cupboard to constant weight at room temperature, followed by lightly milling using a mortar and a pestle and sieving through a 250 µm sieve. Electrostatic charge after one week storage in a dessicator at room temperature: - 24.7 nC/g specific charge and 78.3% transported mass.

Composition of formulation:
0.2200 g Fenoterol hydrobromide, treated with sorbitan trioleate (see hereto above);
4.4880 g Glucose 35 µm;
0.7920 g micronized Glucose;

The components are carefully mixed and filled into capsules or blisters for use in commercial inhaler devices

### Example 2

4 g Tiotropiumbromide monohydrate are dispersed in an incubator in 200 ml of n-hexane containing 3000 mg/L sorbitan trioleate and agitated at 220rpm for 3 hours at 25 ± 0.5°C. The treated drug is filtered using vacuum and dried in a fume cupboard to constant weight at room temperature, followed by lightly milling using a mortar and a pestle and sieving through a 250 µm sieve. Electrostatic charge after one week storage in a dessicator at room temperature: - 96.4 nC/g specific charge and 13.5% transported mass.

Composition of formulation:
0.0225 g Tiotropiumbromide monohydrate, treated with sorbitan trioleate (see hereto above);
5.2036 g Lactose 200 M;
0.2739 g micronized lactose;

The components are carefully mixed and filled into capsules or blisters for use in commercial inhaler devices.

### Example 3

4 g Tiotropiumbromide monohydrate are dispersed in an incubator in 200 ml of n-hexane containing 2000 mg/L sorbitan monostearate and agitated at 220rpm for 3 hours at 25 ± 0.5°C. The treated drug is filtered using vacuum and dried in a fume cupboard to constant weight at room temperature, followed by lightly milling using a mortar and a pestle and sieving through a 250 µm sieve. Electrostatic charge after one week storage in a dessicator at room temperature: - 31.4 nC/g specific charge and 63.7% transported mass.

Composition of formulation:
0.0225 g Tiotropiumbromide monohydrate, treated with sorbitan monostearate (see hereto above);
5.2036 g Lactose 200 M;
0.2739 g micronized lactose;

The components are carefully mixed and filled into capsules or blisters for use in commercial inhaler devices.

### Example 4

4 g Tiotropiumbromide monohydrate are dispersed in an incubator in 200 ml of n-hexane containing 2000 mg/L sorbitan mono-oleate and agitated at 220rpm for 3 hours at 25 ± 0.5°C. The treated drug is filtered using vacuum and dried in a fume cupboard to constant weight at room temperature, followed by lightly milling using a mortar and a pestle and sieving through a 250 µm sieve. Electrostatic charge after one week storage in a dessicator at room temperature: - 31.4 nC/g specific charge and 60.0% transported mass.

Composition of formulation:
0.0225 g Tiotropiumbromide monohydrate, treated with sorbitan mono-oleate (see hereto above);
5.2036 g Lactose 200 M;
0.2739 g micronized lactose;

The components are carefully mixed and filled into capsules or blisters for use in commercial inhaler devices.

### Example 5

4 g Oxitropiumbromide are dispersed in an incubator in 200 ml of n-hexane containing 2000 mg/L sorbitan trioleate and agitated at 220rpm for 3 hours at 25 ± 0.5°C. The treated drug is filtered using vacuum and dried in a fume cupboard to constant weight at room temperature, followed by lightly milling using a mortar and a pestle and sieving through a 250 µm sieve. Electrostatic charge after one week storage in a dessicator at room temperature: 78.7 nC/g specific charge and 33.1% transported mass.

Composition of formulation:
0.11 g Oxitropiumbromide, treated with sorbitan trioleate (see hereto above);
4.5815 g Glucose 35 µm;
0.8085 g micronized glucose;

The components are carefully mixed and filled into capsules or blisters for use in commercial inhaler devices.

### Example 6

4 g Ipratropiumbromide are dispersed in an incubator in 200 ml of n-hexane containing 2000 mg/L sorbitan trioleate and agitated at 220rpm for 3 hours at 25 ± 0.5°C. The treated drug is filtered using vacuum and dried in a fume cupboard to constant weight at room temperature, followed by lightly milling using a mortar and a pestle and sieving through a 250 µm sieve. Electrostatic charge after one week storage in a dessicator at room temperature: 78.2 nC/g specific charge and 34.2% transported mass.

Composition of formulation:
0.2296 g Ipratropiumbromide, treated with sorbitan trioleate (see hereto above);
4.2163 g Glucose 35 µm;
1.0541 g micronized glucose;

The components are carefully mixed and filled into capsules or blisters for use in commercial inhaler devices

### Example 7

10 g of trehalose is dissolved in 50 ml of 20 mM phosphate buffer pH 5.5 containing 0.1% Tween 80 (PEG sorbitan mono-oleate). 50 ml of a solution of 55 mg of Interferon-omega in 20 mM phosphate buffer pH 5.5 is slowly added under gentle stirring. The solution is spray dried at 90°C inlet temperature and 60°C outlet temperature. The almost free flowing powder is easily harvested from the cyclon and dried under vacuum for 6 hours at 40°C. The powder is filled into capsules, but may be diluted by carrier 1:10 prior to filling into the capsules.

### Example 8

10 g of hydroxyethyl starch is dissolved in 100 ml of 20 mM phosphate buffer pH 5.5 containing 0.5% Tween 80 (PEG sorbitan mono-oleate). 100 ml of a solution of 55 mg of Interferon-omega in 20 mM phosphate buffer pH 5.5 is slowly added under gentle stirring. The solution is spray dried at 90°C inlet temperature and 60°C outlet temperature. The powder is harvested from the cyclon and dried under vacuum for 6 hours at 40°C. The powder is filled into capsules, but may be diluted by carrier 1:10 prior to filling into the capsules.

## Claims

1. An improved pharmaceutical dosage form for the use in a dry powder inhalation device (DPI) which comprises
(a) at least one micronized or spray dried solid active ingredient, which is soluble in water and which is selected from among ipratropium, tiotropium, fenoterol and formoterol;
(b) optionally a solid, pharmaceutically acceptable carrier excipient. which dilutes the active ingredient (a);
(c) fatty acid sorbitan ester or a PEG ether thereof;
**characterized in that** the fatty acid sorbitan ester or a PEG ether thereof (c) coats at least partially the surface of (a) or the surface of the agglomerate or mixture formed by (a) and (b).

2. A dosage form as defined in claim 1 obtainable by a process comprising the steps of:
(i) preparation of a solution or dispersion of component (c) in a solvent, in which components (a) and optionally a carrier (b) are insoluble;
(ii) adsorption of component (c) to the surface of (a) and optionally (b) until equilibration;
(iii) separation of the dosage form by filtration and/or centrifugation, and
(iv) optionally drying of the resulting dosage form.

3. A dosage form as defined in claim 2 wherein the solvent of step (i) is a C₃-C₁₂ alkane or a C₃-C₁₂ cycloalkane.

4. A dosage form as defined in claim 3 wherein the solvent of step (i) is a C₅-C₈ alkane or a C₅-C₈ cycloalkane, preferably n-hexane or cyclohexane.

5. A dosage form as defined in claim 1 obtainable by a process comprising the steps of:
(i) dissolving or dispersing components (a) and (c) in a solvent, optionally also adding encapsulating agents (d),
(ii) spray drying the solution or dispersion in a spray dryer under appropriate conditions resulting in microfine particles according to the particle size range described above,
(iii) harvesting the spray dried particles in the cyclone or in the filter,
(iv) optionally drying the particles to reach the wanted moisture content,
(v) and finally optionally diluting the powder by addition of a carrier substance (b).

6. A dosage form as defined in claim 1 obtainable by a process comprising the steps of:
(i) intensively mixing a powder containing the microfine drug substance (a), optionally also drug carrier (b), using standard mixing machines such as a Diosna mixer or a Lödige mixer,
(ii) either adding before start of the mixing process or, preferantially, during the mixing process components (c) to the powder and
(iii) running the mixing process for a while to enable that components (c) coat the surface of components (a) and optionally (b).

7. A dosage form as defined in any of the preceding claims wherein the solid active ingredient (a) is selected from among ipratropium and tiotropium in the form of the chloride, bromide, iodide or methansulfonate thereof.

8. A dosage form as defined in any of the preceding claims wherein the solid active ingredient (a) is selected from among ipratropium bromide and tiotropium bromide.

9. A dosage form as defined in one of claims 1 to 6, wherein the solid active ingredient (a) is selected from among fenoterol, salmeterol and formoterol, optionally in the form of their pharmaceutically acceptable acid addition salts, or in the form of the solvates and hydrates thereof.

10. A dosage form as defined in any of the preceding claims wherein the active ingredient (a) or agglomerates formed by (a) and (b) containing the active ingredient have a mean mass aerodynamic diameter of 0.5 - 8 µm.

11. A dosage form as defined in any of the preceding claims wherein the pharmaceutically acceptable carrier (b) or the pharmaceutically acceptable encapsulating agent or both are a carbohydrate or an inorganic salt

12. A dosage form as defined in claim 11 wherein the pharmaceutically acceptable carrier is selected from lactose and glucose, optionally containing portions of micronized carrier, and the pharmaceutically acceptable encapsulating agent is selected from trehalose, lactose, hydroxyethyl starch, mannitol or mannitol-sucrose, or mixtures thereof.

13. A dosage form as defined in any of the preceding claims 1 to 12 wherein the (c) is selected from the group consisting of sorbitan mono-oleate, sorbitan trioleate, sorbitan monostearate, sorbitan tristearate, sorbitan monolaurate, sorbitan trilaurate, sorbitan monomyristate, sorbitan trimyristate, sorbitan monopalmitate, sorbitan tripalmitate, PEG sorbitan monolaurate, PEG sorbitan monopalmitate, PEG sorbitan monostearate, PEG sorbitan tristearate, PEG sorbitan mono-oleate and PEG sorbitan trioleate.

14. A dosage form as defined in any of the preceding claims the mean specific charge thereof has been reduced for at least 50 % compared with the corresponding sieved active ingredient (a) which has not been treated with (c).

15. A method for the manufacture of the dosage form according to one of claims 1 to 14 which comprises the steps of:
(i) preparation of a solution or dispersion of a fatty acid sorbitan ester or a PEG ether thereof (c) in a solvent, in which the water soluble active ingredient (a), or the agglomerate or mixture formed by (a) and the pharmaceutically acceptable carrier excipient (b) are insoluble;
(ii) adsorption of component (c) to the surface of (a) and optionally (b) until equilibration;
(iii) separation of the dosage form by filtration and/or centrifugation, and
(iv) optionally drying of the resulting dosage form.

16. A method for the manufacture of the dosage form according to one of claims 1 to 14 which comprises the steps of:
(i) dissolving or dispersing components (a) and (c) in a solvent, optionally also adding encapsulating agents (d),
(ii) spray drying the solution or dispersion in a spray dryer under appropriate conditions resulting in microfine particles according to the particle size range described above
(iii) harvesting the spray dried particles in the cyclone or in the filter
(iv) optionally drying the particles to reach the wanted moisture content
(v) and finally optionally diluting the powder by addition of a carrier substance (b).

17. A method for the manufacture of the dosage form according to one of claims 1 to 14 which comprises the steps of:
(i) intensively mixing a powder containing the microfine drug substance (a), optionally also drug carrier (b), using standard mixing machines such as a Diosna mixer or a Lödige mixer,
(ii) either adding before start of the mixing process or, preferantially, during the mixing process components (c) to the powder and
(iii) running the mixing process for a while to enable that components (c) coat the surface of components (a) and optionally (b).

18. A dry powder inhalation device comprising:
(a) manually actuable metered dosing means;
(b) a mouthpiece; and
(c) a housing defining a supply chamber which contains a pharmaceutical dosage form according to any of claims 1 to 14.

## Patentansprüche

1. Verbesserte pharmazeutische Dosierungsform für die Verwendung in einer Trockenpulverinhalationsvorrichtung (DPI), welche umfasst:
(a) mindestens einen mikronisierten oder sprühgetrockneten festen Wirkstoff bzw. aktiven Bestandteil, der in Wasser löslich ist und der ausgewählt ist aus Ipratropium, Tiotropium, Fenoterol und Formoterol;
(b) gegebenenfalls einen festen, pharmazeutisch akzeptablen Trägerhilfsstoff, der den aktiven Bestandteil (a) verdünnt;
(c) Fettsäuresorbitanester oder einen PEG-Ether hiervon;
**dadurch gekennzeichnet, dass** der Fettsäuresorbitanester oder ein PEG-Ether hiervon (c) zumindest teilweise die Oberfläche von (a) oder die Oberfläche des Agglomerates oder der Mischung, gebildet von (a) und (b), beschichtet.

2. Dosierungsform nach Anspruch 1, erhältlich durch ein Verfahren, umfassend die Schritte:
(i) Herstellung einer Lösung oder Dispersion der Komponente (c) in einem Lösungsmittel, in dem die Komponente (a) und gegebenenfalls ein Träger (b) unlöslich sind,
(ii) Adsorption der Komponente (c) an der Oberfläche von (a) und gegebenenfalls (b) bis zur Gleichgewichtseinstellung,
(iii) Abtrennung der Dosierungsform durch Filtrieren und/oder Zentrifugieren, und
(iv) gegebenenfalls Trocknen der resultierenden Dosierungsform.

3. Dosierungsform nach Anspruch 2, wobei das Lösungsmittel von Schritt (i) ein C₃-C₁₂-Alkan oder ein C₃-C₁₂-Cycloalkan darstellt.

4. Dosierungsform nach Anspruch 3, wobei das Lösungsmittel von Schritt (i) ein C₅-C₈-Alkan oder ein C₅-C₈-Cycloalkan darstellt, bevorzugt n-Hexan oder Cyclohexan.

5. Dosierungsform nach Anspruch 1, erhältlich durch ein Verfahren, umfassend die Schritte:
(i) Lösen oder Dispergieren der Komponenten (a) und (c) in einem Lösungsmittel, gegebenenfalls auch unter Zugabe von Ein- bzw. Verkapselungsmitteln (d),
(ii) Sprühtrocknen der Lösung oder Dispersion in einem Sprühtrockner unter geeigneten Bedingungen, woraus gemäß des oben beschriebenen Partikelgrößenbereichs mikrofeine Partikel resultieren,
(iii) Gewinnen der sprühgetrockneten Partikel im Zyklon oder im Filter,
(iv) gegebenenfalls Trocknen der Partikel, um den gewünschten Feuchtigkeitsgehalt zu erreichen,
(v) und schließlich gegebenenfalls Verdünnen des Pulvers durch Zugabe einer Trägersubstanz (b).

6. Dosierungsform nach Anspruch 1, erhältlich durch ein Verfahren, umfassend die Schritte:
(i) intensives Mischen eines Pulvers, enthaltend die mikrofeine Arzneistoffsubstanz (a), gegebenenfalls ebenfalls einen Arzneistoffträger (b), unter Verwendung von Standardmischmaschinen, wie einem Diosna-Mischer oder einem Lödige-Mischer,
(ii) Zugeben der Komponenten (c) zu dem Pulver, entweder vor dem Beginn des Mischverfahrens oder vorzugsweise während des Mischverfahrens, und
(iii) Fortführen des Mischverfahrens für eine Zeit, um zu ermöglichen, dass die Komponenten (c) die Oberfläche der Komponenten (a) und gegebenenfalls (b) beschichten.

7. Dosierungsform nach irgendeinem der vorhergehenden Ansprüche, wobei der feste aktive Bestandteil (a) ausgewählt ist aus Ipratropium und Tiotropium in Form des Chlorids, Bromids, Iodids oder Methansulfonats davon.

8. Dosierungsform nach irgendeinem der vorhergehenden Ansprüche, wobei der feste aktive Bestandteil (a) ausgewählt ist aus Ipratropiumbromid und Tiotropiumbromid.

9. Dosierungsform nach einem der Ansprüche 1 bis 6, wobei der feste aktive Bestandteil (a) ausgewählt ist aus Fenoterol, Salmeterol und Formoterol, gegebenenfalls in Form ihrer pharmazeutisch akzeptablen Säureadditionssalze oder in Form der Solvate und Hydrate hiervon.

10. Dosierungsform nach irgendeinem der vorhergehenden Ansprüche, wobei der aktive Bestandteil (a) oder von (a) und (b) gebildete Agglomerate, enthaltend den aktiven Bestandteil, einen massegemittelten aerodynamischen Durchmesser von 0,5 bis 8 µm aufweisen.

11. Dosierungsform nach irgendeinem der vorhergehenden Ansprüche, wobei der pharmazeutisch akzeptable Träger (b) oder das pharmazeutisch akzeptable Ein- bzw. Verkapselungsmittel oder beide ein Kohlenhydrat oder ein anorganisches Salz darstellen.

12. Dosierungsform nach Anspruch 11, wobei der pharmazeutisch akzeptable Träger ausgewählt ist aus Lactose und Glucose, gegebenenfalls enthaltend Anteile von mikronisiertem Träger, und das pharmazeutisch akzeptable Ein- bzw. Verkapselungsmittel ausgewählt ist aus Trehalose, Lactose, Hydroxyethylstärke, Mannitol oder Mannitolsaccharose oder Mischungen hiervon.

13. Dosierungsform nach irgendeinem der vorhergehenden Ansprüche 1 bis 12, wobei (c) ausgewählt ist aus der Gruppe, bestehend aus Sorbitanmonooleat, Sorbitantrioleat, Sorbitanmonostearat, Sorbitantristearat, Sorbitanmonolaurat, Sorbitantrilaurat, Sorbitanmonomyristat, Sorbitantrimyristat, Sorbitanmonopalmitat, Sorbitantripalmitat, PEG-Sorbitanmonolaurat, PEG-Sorbitanmonopalmitat, PEG-Sorbitanmonostearat, PEG-Sorbitantristearat, PEG-Sorbitanmonooleat und PEG-Sorbitantrioleat.

14. Dosierungsform nach irgendeinem der vorhergehenden Ansprüche, wobei die mittlere spezifische Beladung hiervon um mindestens 50% reduziert wurde, verglichen mit dem entsprechenden gesiebten aktiven Bestandteil (a), der nicht mit (c) behandelt wurde.

15. Verfahren zur Herstellung der Dosierungsform nach einem der Ansprüche 1 bis 14, das die Schritte umfasst:
(i) Herstellung einer Lösung oder Dispersion eines Fettsäuresorbitanesters oder eines PEG-Ethers davon (c) in einem Lösungsmittel, in dem der wasserlösliche aktive Bestandteil (a) oder das Agglomerat oder die Mischung, gebildet durch (a) und den pharmazeutisch akzeptablen Trägerhilfsstoff (b), unlöslich sind;
(ii) Adsorption von Komponente (c) an der Oberfläche von (a) und gegebenenfalls (b) bis zur Gleichgewichtseinstellung;
(iii) Abtrennung der Dosierungsform durch Filtration und/oder Zentrifugieren, und
(iv) gegebenenfalls Trocknen der resultierenden Dosierungsform.

16. Verfahren zur Herstellung der Dosierungsform nach einem der Ansprüche 1 bis 14, das die Schritte umfasst:
(i) Lösen oder Dispergieren der Komponenten (a) und (c) in einem Lösungsmittel, gegebenenfalls auch unter Zugabe von Ein- bzw. Verkapselungsmitteln (d),
(ii) Sprühtrocknen der Lösung oder Dispersion in einem Sprühtrockner unter geeigneten Bedingungen, woraus gemäß des oben beschriebenen Partikelgrößenbereichs mikrofeine Partikel resultieren,
(iii) Gewinnen der sprühgetrockneten Partikel im Zyklon oder im Filter,
(iv) gegebenenfalls Trocknen der Partikel, um den gewünschten Feuchtigkeitsgehalt zu erreichen,
(v) und schließlich gegebenenfalls Verdünnen des Pulvers durch Zugabe einer Trägersubstanz (b).

17. Verfahren zur Herstellung der Dosierungsform nach einem der Ansprüche 1 bis 14, das die Schritte umfasst:
(i) intensives Mischen eines Pulvers, enthaltend die mikrofeine Arzneistoffsubstanz (a), gegebenenfalls ebenfalls einen Arzneistoffträger (b), unter Verwendung von Standardmischmaschinen, wie einem Diosna-Mischer oder einem Lödige-Mischer,
(ii) Zugeben der Komponenten (c) zu dem Pulver, entweder vor dem Beginn des Mischverfahrens oder vorzugsweise während des Mischverfahrens, und
(iii) Fortführen des Mischverfahrens für eine Zeit, um zu ermöglichen, dass die Komponenten (c) die Oberfläche der Komponenten (a) und gegebenenfalls (b) beschichten.

18. Trockenpulverinhalationsvorrichtung, umfassend:
(a) manuell zu betätigendes Dosierungsmittel;
(b) ein Mundstück; und
(c) ein Gehäuse, das eine Vorratskammer definiert, die eine pharmazeutische Dosierungsform nach irgendeinem der Ansprüche 1 bis 14 enthält.

## Revendications

1. Forme galénique pharmaceutique améliorée pour l'utilisation dans un dispositif d'inhalation de poudre sèche (DPI) qui comprend
(a) au moins un principe actif solide micronisé ou séché par pulvérisation, qui est soluble dans l'eau et qui est choisi parmi l'ipratropium, le tiotropium, le fénotérol et le formotérol ;
(b) éventuellement un excipient support solide pharmaceutiquement acceptable qui dilue le principe actif (a) ;
(c) un ester d'acide gras de sorbitane ou un éther de PEG de celui-ci ;
**caractérisée en ce que** l'ester d'acide gras de sorbitane ou un éther de PEG de celui-ci (c) recouvre au moins partiellement la surface de (a) ou la surface de l'agglomérat ou du mélange formé par (a) et (b).

2. Forme galénique telle que définie dans la revendication 1, susceptible d'être obtenue par un procédé comprenant les étapes suivantes :
(i) la préparation d'une solution ou d'une dispersion du composant (c) dans un solvant, dans lequel les composants (a) et éventuellement un support (b) sont insolubles ;
(ii) l'adsorption du composant (c) sur la surface de (a) et éventuellement (b) jusqu'à l'équilibration ;
(iii) la séparation de la forme galénique par filtration et/ou centrifugation, et
(iv) éventuellement le séchage de la forme galénique résultante.

3. Forme galénique telle que définie dans la revendication 2, dans laquelle le solvant de l'étape (i) est un alcane en C₃ à C₁₂ ou un cycloalcane en C₃ à C₁₂.

4. Forme galénique telle que définie dans la revendication 3, dans laquelle le solvant de l'étape (i) est un alcane en C₅ à C₈ ou un cycloalcane en C₅ à C₈, de préférence du n-hexane ou du cyclohexane.

5. Forme galénique telle que définie dans la revendication 1, susceptible d'être obtenue par un procédé comprenant les étapes suivantes :
(i) la dissolution ou la dispersion des composants (a) et (c) dans un solvant, éventuellement également l'addition d'agents d'encapsulation (d),
(ii) le séchage par pulvérisation de la solution ou de la dispersion dans un appareil de séchage par pulvérisation dans des conditions appropriées entraînant des particules microfines selon la plage de tailles des particules décrite ci-dessus,
(iii) la récolte des particules séchées par pulvérisation dans le cyclone ou dans le filtre,
(iv) et éventuellement le séchage des particules pour atteindre la teneur en humidité souhaitée,
(v) et finalement éventuellement la dilution de la poudre par addition d'une substance support (b).

6. Forme galénique telle que définie dans la revendication 1, susceptible d'être obtenue par un procédé comprenant les étapes suivantes :
(i) le mélange intensif d'une poudre contenant la substance médicamenteuse microfine (a), éventuellement également le support de médicament (b), en utilisant des machines de mélange classiques tel qu'un mélangeur Diosna ou un mélangeur Lödige,
(ii) soit l'addition avant le démarrage du procédé de mélange soit, de manière préférentielle, durant le procédé de mélange des composants (c) à la poudre et
(iii) l'exécution du procédé de mélange pendant un certain temps pour permettre aux composants (c) de recouvrir la surface des composants (a) et éventuellement (b).

7. Forme galénique telle que définie dans l'une quelconque des revendications précédentes, dans laquelle le principe actif solide (a) est choisi parmi l'ipratropium et le tiotropium sous la forme du chlorure, du bromure, de l'iodure ou du méthanesulfonate de ceux-ci.

8. Forme galénique telle que définie dans l'une quelconque des revendications précédentes, dans laquelle le principe actif solide (a) est choisi parmi le bromure d'ipratropium et le bromure de tiotropium.

9. Forme galénique telle que définie dans l'une des revendications 1 à 6, dans laquelle le principe actif solide (a) est choisi parmi le fénotérol, le salmétérol et le formotérol, éventuellement sous la forme de leurs sels d'addition d'acide pharmaceutiquement acceptables, ou sous la forme des solvates et des hydrates de ceux-ci.

10. Forme galénique telle que définie dans l'une quelconque des revendications précédentes, dans laquelle le principe actif (a) ou des agglomérats formés par (a) et (b) contenant le principe actif présentent un diamètre aérodynamique moyen en masse de 0,5 à 8 µm.

11. Forme galénique telle que définie dans l'une quelconque des revendications précédentes, dans laquelle le support pharmaceutiquement acceptable (b) ou l'agent d'encapsulation pharmaceutiquement acceptable ou les deux sont un glucide ou un sel inorganique.

12. Forme galénique telle que définie dans la revendication 11, dans laquelle le support pharmaceutiquement acceptable est choisi parmi le lactose et le glucose, contenant éventuellement des parties de support micronisé, et l'agent d'encapsulation pharmaceutiquement acceptable est choisi parmi le tréhalose, le lactose, l'hydroxy-éthyl-amidon, le mannitol ou le mannitolsaccharose, ou des mélanges de ceux-ci.

13. Forme galénique telle que définie dans l'une quelconque des revendications 1 à 12 précédentes, dans laquelle le (c) est choisi dans le groupe constitué par le monooléate de sorbitane, le trioléate de sorbitane, le monostéarate de sorbitane, le tristéarate de sorbitane, le monolaurate de sorbitane, le trilaurate de sorbitane, le monomyristate de sorbitane, le trimyristate de sorbitane, le monopalmitate de sorbitane, le tripalmitate de sorbitane, le monolaurate de PEG sorbitane, le monopalmitate de PEG sorbitane, le monostéarate de PEG sorbitane, le tristéarate de PEG sorbitane, le monooléate de PEG sorbitane et le trioléate de PEG sorbitane.

14. Forme galénique telle que définie dans l'une quelconque des revendications précédentes, dont la charge spécifique moyenne a été réduite d'au moins 50 % comparativement au principe actif (a) tamisé correspondant qui n'a pas été traité avec (c).

15. Méthode de fabrication de la forme galénique selon l'une des revendications 1 à 14, qui comprend les étapes suivantes :
(i) la préparation d'une solution ou d'une dispersion d'un ester d'acide gras de sorbitane ou d'un éther de PEG de celui-ci (c) dans un solvant, dans lequel le principe actif (a) hydrosoluble ou l'agglomérat ou le mélange formé par (a) et l'excipient support pharmaceutiquement acceptable (b) sont insolubles ;
(ii) l'adsorption du composant (c) sur la surface de (a) et éventuellement (b) jusqu'à l'équilibration ;
(iii) la séparation de la forme galénique par filtration et/ou centrifugation, et
(iv) éventuellement le séchage de la forme galénique résultante.

16. Méthode de fabrication de la forme galénique selon l'une des revendications 1 à 14 qui comprend les étapes suivantes :
(i) la dissolution ou la dispersion des composants (a) et (c) dans un solvant, éventuellement également l'addition d'agents d'encapsulation (d),
(ii) le séchage par pulvérisation de la solution ou de la dispersion dans un appareil de séchage par pulvérisation dans des conditions appropriées entraînant des particules microfines selon la plage de tailles des particules décrite ci-dessus,
(iii) la récolte des particules séchées par pulvérisation dans le cyclone ou dans le filtre,
(iv) éventuellement le séchage des particules pour atteindre la teneur en humidité souhaitée,
(v) et finalement éventuellement la dilution de la poudre par addition d'une substance support (b).

17. Méthode de fabrication de la forme galénique selon l'une des revendications 1 à 14, qui comprend les étapes suivantes :
(i) le mélange intensif d'une poudre contenant la substance médicamenteuse microfine (a), éventuellement également le support de médicament (b), en utilisant des machines de mélange classiques tel qu'un mélangeur Diosna ou un mélangeur Lödige,
ii) soit l'addition avant le démarrage du procédé de mélange soit, de manière préférentielle, durant le procédé de mélange des composants (c) à la poudre et
(iii) l'exécution du procédé de mélange pendant un certain temps pour permettre aux composants (c) de recouvrit surface des composants (a) et éventuellement (b).

18. Dispositif d'inhalation de poudre sèche comprenant :
(a) un moyen de dosages pouvant être actionné manuellement ;
(b) un embout buccal ; et
(c) un logement définissant une chambre d'alimentation qui contient une forme galénique pharmacetique selon l'une quelconque des revendications 1 à 14.
